(19) Europäisches Patentamt
European Patent Office
Office européen des brevets

(11) Veröffentlichungsnummer : **0 348 736 B1**

(12) # EUROPÄISCHE PATENTSCHRIFT

(45) Veröffentlichungstag der Patentschrift : **11.11.92 Patentblatt 92/46**

(51) Int. Cl.[5] : **C07D 285/12**, A01N 43/82, C07D 417/12

(21) Anmeldenummer : **89110798.9**

(22) Anmeldetag : **14.06.89**

(54) **Substituierte Thiadiazolyloxyessigsäureamide.**

Die Akte enthält technische Angaben, die nach dem Eingang der Anmeldung eingereicht wurden und die nicht in dieser Patentschrift enthalten sind.

(30) Priorität : **27.06.88 DE 3821599**

(43) Veröffentlichungstag der Anmeldung : **03.01.90 Patentblatt 90/01**

(45) Bekanntmachung des Hinweises auf die Patenterteilung : **11.11.92 Patentblatt 92/46**

(84) Benannte Vertragsstaaten : **BE CH DE FR GB IT LI NL**

(56) Entgegenhaltungen :
**EP-A- 0 217 496**
**US-A- 4 005 213**

(73) Patentinhaber : **BAYER AG**
**W-5090 Leverkusen 1 Bayerwerk (DE)**

(72) Erfinder : **Förster, Heinz, Dr.**
**Am Eckbusch 47**
**W-5600 Wuppertal 1 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch Gladbach 2 (DE)**
Erfinder : **Strang, Harry, Dr.**
**Unterdorfstrasse 6A**
**W-4000 Düsseldorf 31 (DE)**

**EP 0 348 736 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Thiadiazolyloxyessigsäureamide, ein Verfahren und neue Zwischenprodukte zu ihrer Herstellung sowie ihre Verwendung als Herbizide.

Es ist bereits bekannt, daß bestimmte Heteroaryloxyessigsäureamide, wie z. B. 2-(Benzthiazol-2-yl-oxy)-essigsäure-N-methyl-anilid herbizide Eigenschaften aufweisen (vgl, US-P 4 509 971).

Ferner ist bekannt, daß auch bestimmte, in 5-Stellung durch eine $RXCF_2$-Gruppe substituierte 1,3,4-Thiadiazolyloxyessigsäureamide herbizid wirksam sind; hierbei steht, wenn X Sauerstoff bedeutet, R immer für gegebenenfalls substituiertes Phenyl (vgl, EP-A-0 217 496),

Die herbizide Wirksamkeit der vorbekannten Verbindungen ist jedoch nicht immer ganz zufriedenstellend.

Es wurden nun neue substituierte Thiadiazolyloxyessigsäureamide der allgemeinen Formel (I) gefunden,

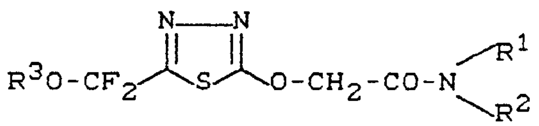

(I)

in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist, und

$R^3$ für $C_1$-$C_6$-Alkyl steht, welches gegebenenfalls durch Halogen, $C_1$-$C_4$-Alkoxy, $C_1$-$C_4$-Alkylsulfonyl, Phenyl, Phenoxy oder Phenyl-$C_1$-$C_4$-alkoxy substituiert ist, wobei die Phenylkomponente gegebenenfalls durch Halogen, $C_1$-$C_4$-Alkyl, Trifluormethyl und/oder $C_1$-$C_4$-Alkoxy substituiert ist.

Weiter wurde gefunden, daß man die neuen substituierten Thiadiazolyloxyessigsäureamide der allgemeinen Formel (I) erhält, wenn man Methylsulfonylthiadiazole der allgemeinen Formel (II)

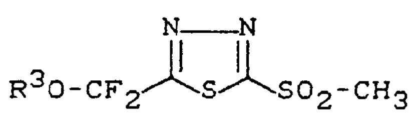

(II)

in welcher

$R^3$ die oben angegebene Bedeutung hat,

mit Hydroxyessigsäureamiden der allgemeinen Formel (III)

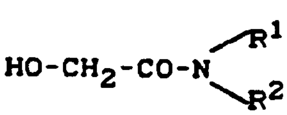

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen substituierten Thiadiazolyloxyessigsäureamide der allgemeinen Formel (I) interessante herbizide Eigenschaften besitzen.

Überraschenderweise zeigen die neuen substituierten Thiadiazolyl-oxyessigsäureamide der allgemeinen Formel (I) erheblich stärkere herbizide Wirkung gegen verbreitete, schwer bekämpfbare Unkräuter als die oben genannte Verbindung bei guter Verträglichkeit gegenüber wichtigen Kulturpflanzen.

Gegenstand der Erfindung sind vorzugsweise Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy und/oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydro-chinolinyl stehen, und

$R^3$ für Methyl, Ethyl, Propyl, Isopropyl, Butyl, Isobutyl, sec-Butyl, tert-Butyl, Trifluorethyl, Tri-chlorethyl, Methoxyethyl, Ethoxyethyl, Methylsulfonylethyl, Ethylsulfonylethyl, Benzyl, Chlorbenzyl, Phenylethyl oder Benzyloxyethyl steht.

Beispiele für die Verbindungen der Formel (I) sind in der nachstehenden Tabelle 1 aufgeführt.

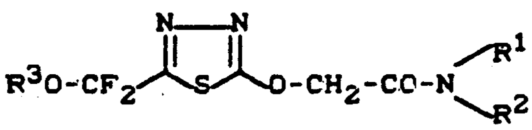

(I)

**Tabelle 1:** Beispiele für die Verbindungen der Formel (I)

| $R^3$ | $R^1$ | $R^2$ | bzw. 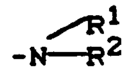 |
|---|---|---|---|
| $CH_3$ | $CH_3$ | $CH_3$ | |
| $CH_3$ | $C_2H_5$ | $C_2H_5$ | |
| $CH_3$ | $C_3H_7$ | $C_3H_7$ | |
| $CH_3$ | $C_4H_9$ | $C_4H_9$ | |
| $CH_3$ | $CH_3$ | $C_4H_9$ | |
| $CH_3$ | $CH_3$ | $CHC_2H_5$<br>\|<br>$CH_3$ | |
| $CH_3$ | $CH_2CH=CH_2$ | $CH_2CH=CH_2$ | |
| $CH_3$ | $CH_2C≡CH$ | $CH_2C≡CH$ | |
| $CH_3$ | $CHC_2H_5$<br>\|<br>$CH_3$ | $OCH_3$ | |
| $CH_3$ | $CH(CH_3)_2$ | $OCH(CH_3)_2$ | |
| $CH_3$ | $CH(CH_3)_2$ | $OCH_2CH_2OC_2H_5$ | |
| $CH_3$ | $CH_3$ | $C_5H_{11}$ | |
| $CH_3$ | $CH_3$ | $C_6H_{13}$ | |
| $CH_3$ | $CH_3$ | 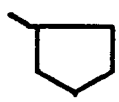 | |

Tabelle 1 - Fortsetzung

| $R^3$ | $R^1$ | $R^2$ | bzw. 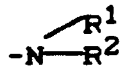 |
|---|---|---|---|
| $CH_3$ | $CH_3$ | 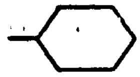 | |
| $CH_3$ | $CH_3$ | 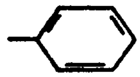 | |
| $CH_3$ | $CH_3$ | 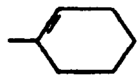 | |
| $CH_3$ | $CH_3$ | 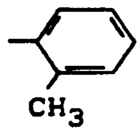 | |
| $CH_3$ | $CH_3$ | 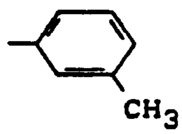 | |
| $CH_3$ | $CH_3$ | 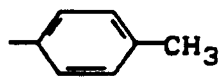 | |
| $CH_3$ | $CH_3$ | 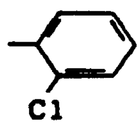 | |
| $CH_3$ | $CH_3$ | 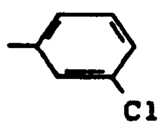 | |
| $CH_3$ | $CH_3$ | 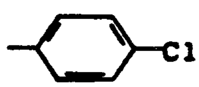 | |

Tabelle 1 - Fortsetzung

| $R^3$ | $R^1$ | $R^2$ | bzw. 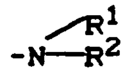 |
|---|---|---|---|
| $CH_3$ | $CH_3$ | 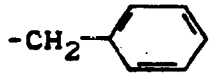 | |
| $CH_3$ | $CH_3$ | 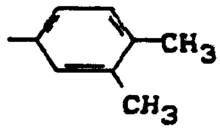 | |
| $CH_3$ | $CH_3$ | 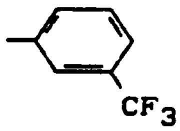 | |
| $CH_3$ | $CH_3$ | 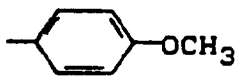 | |
| $CH_3$ | | | 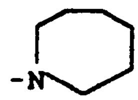 |
| $CH_3$ | | | 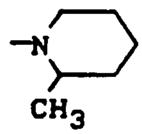 |
| $CH_3$ | | | 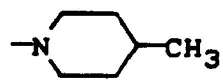 |
| $CH_3$ | | | 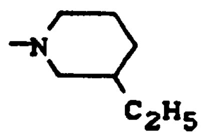 |
| $CH_3$ | | | 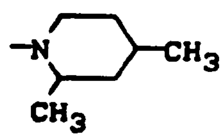 |

Tabelle 1 - Fortsetzung

| $R^3$ | $R^1$ | $R^2$ | bzw. $-N<^{R^1}_{R^2}$ |
|---|---|---|---|
| $CH_3$ | | | 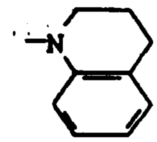 |
| $CH_3$ | $C_2H_5$ | 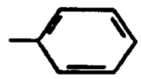 | |
| $CH_3$ | $CH(CH_3)_2$ | 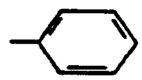 | |
| $CH_3$ | $CH(CH_3)_2$ | 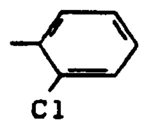 | |
| $CH_3$ | $CH(CH_3)_2$ | 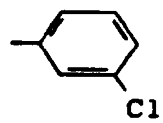 | |
| $CH_3$ | $CH(CH_3)_2$ | 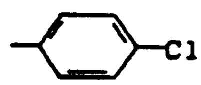 | |
| $CH_3$ | $CH(CH_3)_2$ | 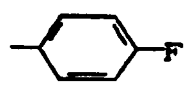 | |
| $CH_3$ | $CH(CH_3)_2$ | 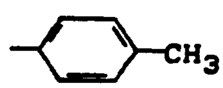 | |
| $CH_3$ | $CH(CH_3)_2$ | 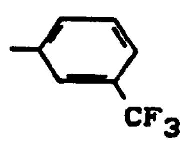 | |

Tabelle 1 - Fortsetzung

| $R^3$ | $R^1$ | $R^2$ | bzw. 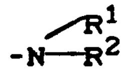 |
|---|---|---|---|
| $CH_3$ | $C_3H_7$ | 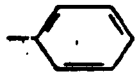 | |
| $C_2H_5$ | $CH_3$ | $C_4H_9$ | |
| $C_2H_5$ | $C_2H_5$ | $C_2H_5$ | |
| $C_2H_5$ | $CH(CH_3)_2$ | $CH_2CH_2OC_2H_5$ | |
| $C_2H_5$ | $CH_3$ | 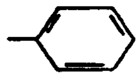 | |
| $C_2H_5$ | $CH(CH_3)_2$ | 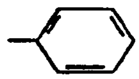 | |
| $C_3H_7$ | $CH_3$ | 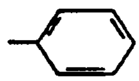 | |
| $C_3H_7$ | $CH(CH_3)_2$ | 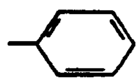 | |
| $C_3H_7$ | $C_3H_7$ | $C_3H_7$ | |
| $CH(CH_3)_2$ | $C_2H_5$ | $C_2H_5$ | |
| $CH(CH_3)_2$ | $CH_3$ | 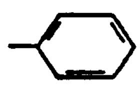 | |
| $CH(CH_3)_2$ | $CH(CH_3)_2$ | 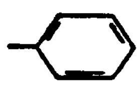 | |
| $C_4H_9$ | $CH_3$ | 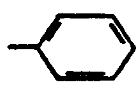 | |

**Tabelle 1 - Fortsetzung**

| $R^3$ | $R^1$ | $R^2$ | bzw. 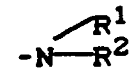 |
|---|---|---|---|
| $CH_2CH_2OCH_3$ | $CH_3$ | 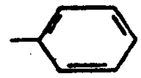 | |
| $CH_2CH_2OCH_3$ | $C_2H_5$ | $C_2H_5$ | |
| $CH_2CH_2OC_2H_5$ | $CH_3$ | 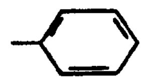 | |
| $CH_2$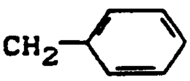 | $CH_3$ | 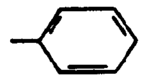 | |
| $CH_2CH_2OCH_2$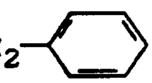 | $CH_3$ | 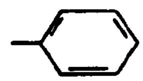 | |
| $CH_2CF_3$ | $CH_3$ | 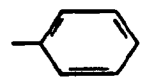 | |

Verwendet man beispielsweise 2-Methylsulfonyl-5-methoxydifluormethyl-1,3,4-thiadiazol und Hydroxyessigsäurepiperidid als Ausgangsstoffe, so läßt sich der Reaktionsablauf beim erfindungsgemäßen Verfahren durch das folgende Formelschema darstellen:

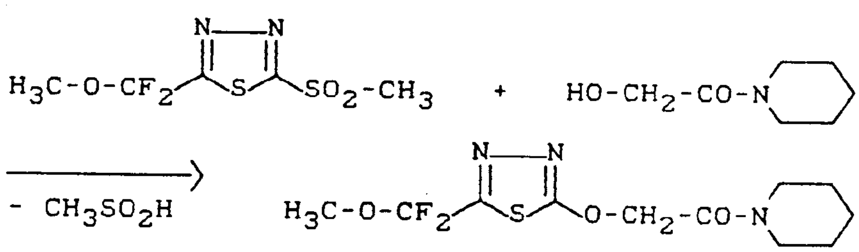


Die beim erfindungsgemäßen Verfahren zur Herstellung von Verbindungen der Formel (I) als Ausgangsstoffe zu verwendenden Methylsulfonylthiadiazole sind durch die Formel (II) allgemein definiert.

In Formel (II) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurde.

Als Beispiele für die Ausgangsstoffe der Formel (II) seien genannt:

5-Methoxy-difluormethyl-, 5-Ethoxydifluormethyl-, 5-Propoxydifluormethyl-, 5-Isopropoxydifluormethyl-, 5-Butoxydifluormethyl-, 5-Isobutoxydifluormethyl-, 5-sec-Butoxydifluormethyl-, 5-tert-Butoxydifluormethyl-, 5(2,2,2-Trifluorethoxy)-difluormethyl-, 5-(2,2,2-Trichlorethoxy)-difluormethyl-, 5-(2-Methoxyethoxy)-difluormethyl-, 5-(2-Ethoxyethoxy)-difluormethyl-, 5-(2-Methylsulfonylethoxy)-difluormethyl-, 5-(2-Ethylsulfonylethoxy)-difluormethyl-, 5-Benzyloxydifluormethyl-, 5-(1-Phenylethoxy)-difluormethyl-, 5-(2-Phenylethoxy)-difluormethyl-, 5-(2-Chlorbenzyloxy)-difluormethyl-, 5-(3-Chlor-benzyloxy)-difluormethyl-, 5-(4-Chlor-benzyloxy)-difluormethyl- und 5-(2-Benzyloxy-ethoxy)-difluormethyl-2-methylsulfonyl-1,3,4-thiadiazol.

Die Ausgangsstoffe der Formel (II) sind noch nicht aus der Literatur bekannt und sind Gegenstand der vorliegenden Erfindung.

Man erhält die neuen Verbindungen der Formel (II), wenn man Methylthio-thiadiazole der allgemeinen For-

mel (IV)

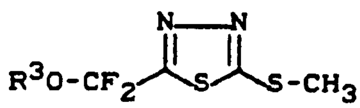

(IV)

in welcher

$R^3$ die oben angegebene Bedeutung hat,

mit einem Oxidationsmittel, wie z. B. Hydrogenperoxid, gegebenenfalls in Gegenwart eines Katalysators, wie z. B. Natriumwolframat, und gegebenenfalls in Gegenwart von Verdünnungsmitteln, wie z.B. Wasser, Ameisensäure und/oder Essigsäure, bei Temperaturen zwischen 0 °C und 100 °C umsetzt.

Die als Zwischenprodukte benötigten Methylthiothiadiazole sind durch die Formel (IV) allgemein definiert. In Formel (IV) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurde.

Als Beispiele für die Zwischenprodukte der Formel (IV) seien genannt:

5-Methoxy-difluormethyl-, 5-Ethoxydifluormethyl-, 5-Propoxydifluormethyl-, 5-Isopropoxydifluormethyl-, 5-Butoxydifluormethyl-, 5-Isobutoxydifluormethyl-, 5-sec-Butoxydifluormethyl-, 5-tert-Butoxydifluormethyl-, 5-(2,2,2-Trifluorethoxy)-difluormethyl-, 5-(2,2,2-Trichlorethoxy)-difluormethyl-, 5-(2-Methoxyethoxy)-difluormethyl-, 5-(2-Ethoxyethoxy)-difluormethyl-, 5-(2-Methylsulfonylethoxy)-difluormethyl-, 5-(2-Ethylsulfonylethoxy)-difluormethyl-, 5-Benzyloxydifluormethyl-, 5-(1-Phenylethoxy)-difluormethyl-, 5-(2-Phenylethoxy)-difluormethyl-, 5-(2-Chlor-benzyloxy)-difluormethyl-, 5-(3-Chlor-benzyloxy)-difluormethyl-, 5-(4-Chlor-benzyloxy)-difluormethyl- und 5-(2-Benzyloxy-ethoxy)-difluormethyl-2-methylthio-1,3,4-thiadiazol.

Die Zwischenprodukte der Formel (IV) sind noch nicht aus der Literatur bekannt und sind Gegenstand der vorliegenden Erfindung.

Man erhält die neuen Verbindungen der Formel (IV), wenn man 5-Chlordifluormethyl-2-methylthio-1,3,4-thiadiazol der Formel (V)

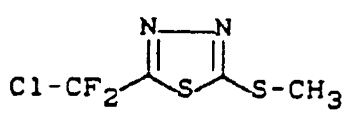

(V)

mit Hydroxyverbindungen der allgemeinen formel (VI)

$$R^3 - OH \qquad (VI)$$

in welcher

$R^3$ die oben angegebene Bedeutung hat,

oder mit deren Natrium- oder Kaliumsalzen gegebenenfalls in Gegenwart von Verdünnungsmitteln, wie z. B. Aceton, Acetonitril oder Toluol bei Temperaturen zwichen 0°C und 50°C umsetzt.

5-Chlordifluormethyl-2-methylthio-1,3,4-thiadiazol der Formel (V) ist noch nicht explizit aus der Literatur bekannt.

Man erhält die Verbindung der formel (V), wenn man Chlordifluoressigsäure mit Dithiocarbazinsäuremethylester in Gegenwart von Phosphoroxychlorid und gegebenenfalls in Gegenwart eines Säureakzeptors, wie z. B. Kaliumcarbonat, sowie gegebenenfalls in Gegenwart eines Verdünnungsmittels, wie z. B. Toluol, bei Temperaturen zwischen 0 °C und 100 °C umsetzt.

Die weiter als Ausgangsstoffe zu verwendenden Hydroxyessigsäureamide sind durch die Formel (III) allgemein definiert. In Formel (III) haben $R^1$ und $R^2$ vorzugsweise diejenigen Bedeutungen, die bereits oben in Rahmen der Beschreibung der erfindungsgemäßen Verbindungen der Formel (I) als bevorzugt für $R^1$ bzw. $R^2$ angegeben wurden.

Beispiele für die Ausgangsstoffe der Formel (III) sind in der nachstehenden Tabelle 2 aufgeführt.

<u>Tabelle 2</u>: Beispiele für die Ausgangsstoffe der Formel (III)

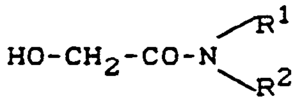 (III)

| $R^1$ | $R^2$ | $R^1$ | $R^2$ |
|---|---|---|---|
| $CH_3$ | $OCH(CH_3)_2$ | $CH_3$ | $OCH_2CH_2OC_2H_5$ |
| $CH_3$ | $CH(CH_3)_2$ | $CH_3$ | $CH_2CH(CH_3)_2$ |
| $CH_3$ | 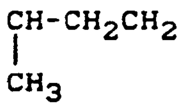 | $C_2H_5$ | $OC_2H_5$ |
| $C_2H_5$ | $OCH_2CH_2OC_2H_5$ | $C_2H_5$ | $OCH_2CH(CH_3)_2$ |

Tabelle 2 - Fortsetzung

| $R^1$ | $R^2$ | $R^1$ | $R^2$ |
|---|---|---|---|
| $CH_3$ | 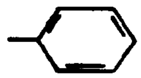 | $CH_3$ | 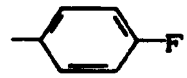 |
| $CH_3$ | 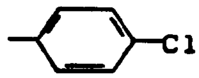 | $CH_3$ | 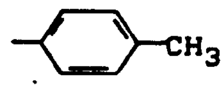 |
| $CH_3$ | 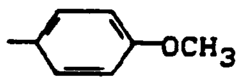 | $CH_3$ | 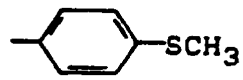 |
| $CH_3$ | 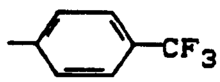 | $CH_3$ | 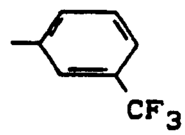 |
| $CH_3$ | 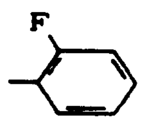 | $CH_3$ | 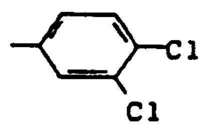 |
| $CH_3$ | 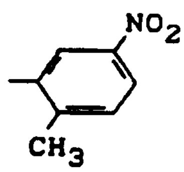 | $C_2H_5$ | 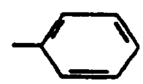 |
| $CH_3$ | 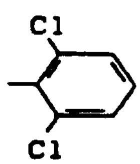 | $CH_3$ | 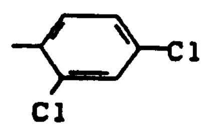 |
| $CH_3$ | 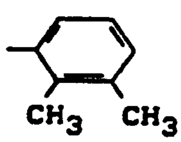 | $CH_3$ | 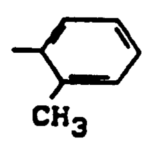 |
| $CH_3$ | 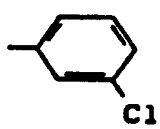 | $CH_3$ | 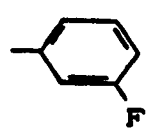 |

**Tabelle 2** - Fortsetzung

| $R^1$ | $R^2$ | $R^1$ | $R^2$ |
|---|---|---|---|
| $CH_3$ | 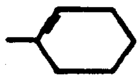 | $CH_3$ | $CH_3$ |
| $CH(CH_3)_2$ | 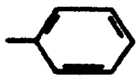 | $C_2H_5$ | 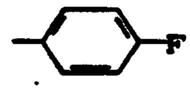 |
| $C_2H_5$ | 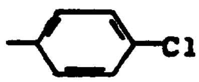 | $C_2H_5$ | 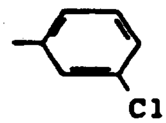 |
| $CH(CH_3)_2$ | 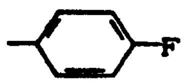 | $CH(CH_3)_2$ | 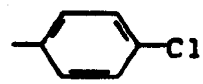 |
| $CH(CH_3)_2$ | 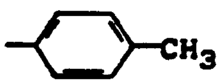 | $CH(CH_3)_2$ | 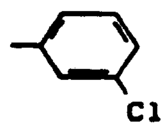 |
| $CH(CH_3)_2$ | 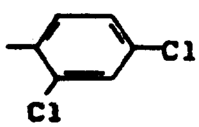 | $CH(CH_3)_2$ | 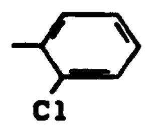 |
| $C_2H_5$ | $C_2H_5$ | $C_3H_7$ | $C_3H_7$ |
| $CH(CH_3)_2$ | $CH(CH_3)_2$ | $CH(CH_3)_2$ | $OCH_3$ |
| $CH(CH_3)_2$ | $OCH_2CH_2OC_2H_5$ | $CH(CH_3)_2$ | $OC_2H_5$ |
| $CH(CH_3)_2$ | $OC_3H_7$ | $CH_3$ | $OCH_2CH(CH_3)_2$ |
| $CH_3$ | $CH_2OCH_3$ | $C_4H_9$ | $C_4H_9$ |
| $C_2H_5$ | $OCH_3$ | $CH_3$ | $CH_2CF_3$ |
| $CH_2CH=CH_2$ | $CH_2CH=CH_2$ | $CH_2C≡CH$ | $CH_2C≡CH$ |
| $CH_3$ | $CH_2C≡CH$ | $CH(CH_3)CH_2CH_3$ | $OCH_3$ |

Tabelle 2 - Fortsetzung

| $R^1$ | $R^2$ | $R^1$ | $R^2$ |
|---|---|---|---|
| 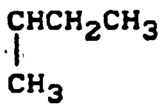 | $OCH_3$ | $CH_3$ | 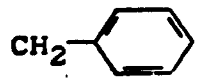 |
| $C_2H_5$ | 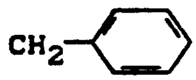 | $CH(CH_3)_2$ | 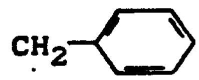 |
| $C_2H_5$ | $CH_2C{\equiv}CH$ | $CH(CH_3)_2$ | $CH_2C{\equiv}CH$ |
| $CH(CH_3)_2$ | $OCH(CH_3)_2$ | | |

| 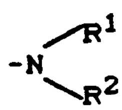 | 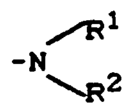 |
|---|---|
| 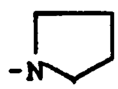 | 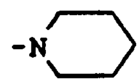 |
| 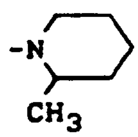 | 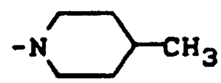 |
| 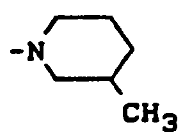 | 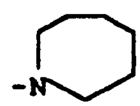 |
| 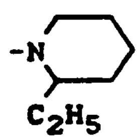 | 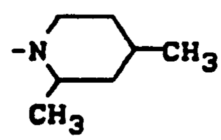 |
| 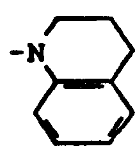 | |

Die Hydroxyessigsäureamide der Formel (III) sind bekannt und/oder können nach an sich bekannten Verfahren hergestellt werden (vgl. US-P 4 509 971 und US-P 4 645 525; ferner US-P 4 334 073, DE-A 3 038 598, DE-A 3 038 636, EP-A 37 526).

Das erfindungsgemäße Verfahren zur Herstellung der neuen substituierten Thiadiazolyloxyessigsäureamide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether,

Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt.Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciumhydroxid und/oder Carbonate, wie z.B. Natrium-, Kalium-, Magnesium- und Calciumcarbonat verwendet.

Der Zusatz von 0,01 bis 10 Gew.-% (bezogen auf eingesetztes Glycolsäureamid der Formel (III) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Methylsulfonylthiadiazol der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in monokotylen und dikotylen Kulturen, vor allem im Vorauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch Chloressigsäure-N-(methoxymethyl)-2,6-diethylanilid (ALACHLOR); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäure methylester (BENSULFURON); N-(Butoxymethyl)-2-chlor-N-(2,6-diethylphenyl)-acetamid (BUTACHLOR); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); exo-1-Methyl-4-(1-methylethyl)-2-(2-methylphenyl-methoxy)-7-oxabicyclo-(2,2,1)-heptan (CINMETHYLIN); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); S-Ethyl-N,N-hexamethylen-thiolcarbamat (MOLINATE); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); α-Chlor-2',6'-diethyl-N-(2-propoxyethyl)-acetanilid (PRETILACHLOR); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

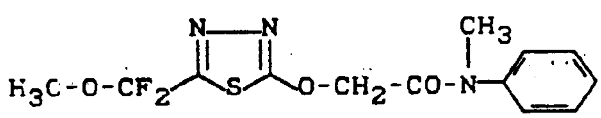

Eine Lösung von 0,88 g (0,022 Mol) Natriumhydroxid in 4 ml Wasser wird unter Rühren zu einer auf -20 °C abgekühlten Mischung aus 4,0 g (0,0189 Mol) 5-Methoxydifluormethyl-2-methylsulfonyl-1,3,4-thiadiazol, 3,1 g (0,0189 Mol) Hydroxyessigsäure-N-methyl-anilid und 40 ml Aceton tropfenweise gegeben. Das Reaktionsgemisch wird 12 Stunden unter Kühlen mit Eis/Kochsalz gerührt. Dann wird mit Essigsäure angesäuert und im Wasserstrahlvakuum eingeengt. Der Rückstand wird mit Wasser/Toluol geschüttelt, die organische Phase mit Wasser gewaschen, mit Natriumsulfat getrocknet und filtriert. Vom Filtrat wird das Lösungsmittel im Wasserstrahlvakuum sorgfältig abdestilliert.

Man erhält 5,8 g (93 % der Theorie) (5-Methoxy-difluormethyl-1,3,4-thiadiazol-2-yl)-oxyessigsäure-N-methylanilid als öligen Rückstand vom

Brechungsindex $n_D^{20} = 1,5350$.

Analog Beispiel 1 und entsprechend der allgemeinen Beschreibung des erfindungsgemäßen Herstellungsverfahrens können auch die in der nachstehenden Tabelle 3 aufgeführten Verbindungen der Formel (I) hergestellt werden:

**Tabelle 3:** Herstellungsbeispiele für die Verbindungen der Formel (I)

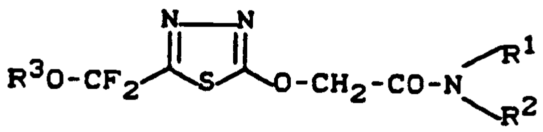 (I)

| Bsp.-Nr. | $R^3$ | $R^1$ | $R^2$ | physikalische Daten |
|---|---|---|---|---|
| | | bzw. 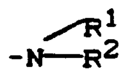 | | |
| 2 | $CH_3$ | $CH(CH_3)_2$ | 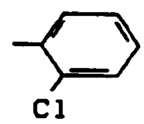 | Fp: 51 °C |
| 3 | $CH_3$ | $CH(CH_3)_2$ | 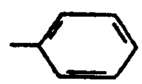 | Fp: 83 °C |
| 4 | $CH_3$ | $CH(CH_3)_2$ | $OCH_2CH_2OC_2H_5$ | $n_D^{20}$: 1,4640 |
| 5 | $CH_3$ | $CH_3$ | 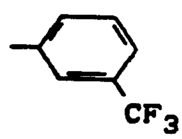 | $n_D^{20}$: 1,4980 |
| 6 | $CH_3$ | $CH_3$ | 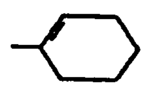 | $n_D^{20}$: 1,4930 |
| 7 | $CH_3$ | 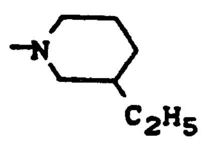 | | $n_D^{20}$: 1,4895 |

Tabelle 3 - Fortsetzung

| Bsp.-Nr. | $R^3$ | $R^1$ bzw. 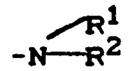 | $R^2$ | physikalische Daten |
|---|---|---|---|---|
| 8 | $CH_3$ | $CH_3$ | $C_4H_9$ | $n_D^{20}$: 1,4750 |
| 9 | $CH_3$ | 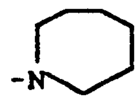 | | $n_D^{20}$: 1,4930 |
| 10 | $CH_3$ | $CH_2CH=CH_2$ | $CH_2CH=CH_2$ | $n_D^{20}$: 1,4860 |
| 11 | $CH_3$ | $CH(CH_3)_2$ | 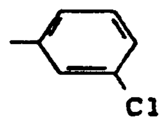 | $n_D^{20}$: 1,5289 |
| 12 | $CH_3$ | $CH(CH_3)_2$ | 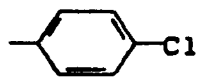 | Fp: 108 °C |
| 13 | $CH_3$ | $C_3H_7$ | $C_3H_7$ | $n_D^{20}$: 1,4745 |
| 14 | $CH_3$ | $CH_3$ | 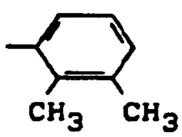 | Fp: 96 °C |
| 15 | $CH_3$ | $CH_3$ | $CH_2C\equiv CH$ | $n_D^{20}$: 1,4923 |

Ausgangsstoffe der Formel (II)

Beispiel (II-1)

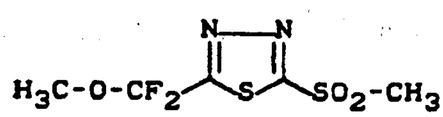

200 ml 35 %ige wäßrige Hydrogenperoxidlösung werden unter Rühren zu einer auf 50 °C erwärmten Mischung aus 40 g (0,16 Mol) 5-Methoxydifluormethyl-2-methylthio-1,3,4-thiadiazol, 0,2 g Natriumwolframat und 140 ml Ameisensäure tropfenweise gegeben, wobei die Temperatur kurzzeitig auf 95 °C steigt. Dann wird das Reaktionsgemisch auf 0 °C abgekühlt und das kristallin angefallene Produkt durch Absaugen isoliert.

Man erhält 22 g (56 % der Theorie) 5-Methoxydifluormethyl-2-methylsulfonyl-1,3,4-thiadiazol vom Schmelzpunkt 45 °C.

Ausgangsstoffe der Formel (IV)

Beispiel (IV-1)

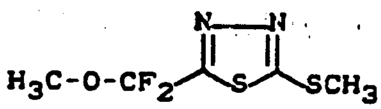

Eine Lösung von 61 g (1,13 Mol) Natriummethylat in 500 ml Methanol wird unter Rühren zu einer auf 0 °C bis 5 °C abgekühlten Lösung von 245 g (0,9 Mol) 5-Chlordifluormethyl-2-methylthio-1,3,4-thiadiazol in 600 ml Methanol tropfenweise gegeben. Das Reaktionsgemisch wird 12 Stunden bei 20 °C gerührt, dann in 2 l Wasser gegossen und mit 2 l Toluol geschüttelt. Die organische Phase wird abgetrennt, mit Natriumsulfat getrocknet und filtriert. Das Filtrat wird im Wasserstrahlvakuum eingeengt und der Rückstand im Hochvakuum destilliert.

Man erhält 95 g (50 % der Theorie) 5-Methoxy-difluormethyl-2-methylthio-1,3,4-thiadiazol als farbloses Öl vom Siedepunkt 68 °C/0,01 mbar.

Ausgangsverbindung der Formel (V)

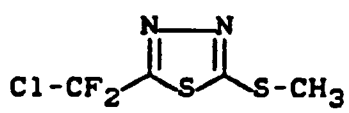

130 g (1,0 Mol) Chlordifluoressigsäure werden bei 20 °C zu einer Lösung von 69 g (0,5 Mol) Kaliumcarbonat in 100 ml Wasser tropfenweise gegeben. Nach Zugabe von 1,5 l Toluol wird das Wasser am Wasserabscheider entfernt. Zu der verbleibenden Suspension werden unter Außenkühlung bei ca. 25 °C 121 g (1,0 Mol) Dithiocarbazinsäuremethylester gegeben und dann werden 195 g (1,25 Mol) Phosphoroxychlorid unter Halten der Temperatur bei 50 °C bis 60 °C zugetropft. Nach zweistündigem Rühren wird die Reaktionsmischung in Eiswasser gegossen, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 190 g (88 % der Theorie) 5-Chlor-difluormethyl-2-methylthio-1,3,4-thiadiazol als öligen Rückstand, welcher durch Destillation im Hochvakuum gereinigt werden kann. Kp: 90 °C - 95 °C/1 mbar.

Anwendungsbeispiel

Im folgenden Anwendungsbeispiel wird die Verbindung nachstehender Formel als Vergleichssubstanz verwendet:

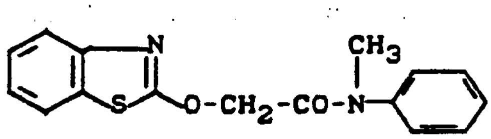

. (A)

2-(Benzthiazol-2-yl-oxy)-essigsäure-N-methyl-anilid (bekannt aus US-P 4 509 971).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß den Herstellungsbeispielen (1), (3), (4), (5), (6), (7), (9), (10), (11), (13) und (14).

## Patentansprüche

1. Substituierte Thiadiazolyloxyessiysäureamide der allgemeinen Formel (I)

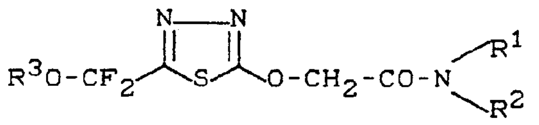

(I)

in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$-oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist, und

$R^3$ für $C_1$-$C_6$-Alkyl steht, welches gegebenenfalls durch Halogen, $C_1$-$C_4$-Alkoxy, $C_1$-$C_4$-Alkylsulfonyl, Phenyl, Phenoxy oder Phenyl-$C_1$-$C_4$-alkoxy substituiert ist, wobei die Phenylkomponente gegebenenfalls durch Halogen, $C_1$-$C_4$-Alkyl, Trifluormethyl und/oder $C_1$-$C_4$-Alkoxy substituiert ist.

2. Verbindungen der Formel (I) gemäß Anspruch 1, dadurch gekennzeichnet, daß darin

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy und/oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydrochinolinyl stehen, und

$R^3$ für Methyl, Ethyl, Propyl, Isopropyl, Butyl, Isobutyl, sec-Butyl, tert-Butyl, Trifluorethyl, Trichlorethyl, Methoxyethyl, Ethoxyethyl, Methylsulfonylethyl, Ethylsulfonylethyl, Benzyl, Chlorbenzyl, Phenylethyl oder Benzyloxyethyl steht.

3. Verfahren zur Herstellung von substituierten Thiadiazolyloxyessigsäureamiden der Formel (I) gemäß Anspruch 1, dadurch gekennzeichnet, daß man Methylsulfonylthiadiazole der allgemeinen Formel (II)

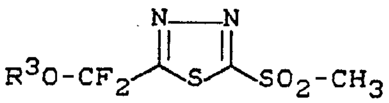

(II)

in welcher

$R^3$ die in Anspruch 1 angegebene Bedeutung hat, mit Hydroxyessigsäureamiden der allgemeinen Formel (III)

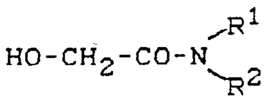

(III)

in welcher

$R^1$ und $R^2$ die in Anspruch 1 angegebenen Bedeutungen haben,

gegebenenfalls in Gegenwart eines Verdünnungs-mittels, gegebenenfalls in Gegenwart eines Säurebindemittels und gegebenenfalls in Gegenwart eines Katalysators umsetzt.

4. Herbizide Mittel gekennzeichnet durch einen Gehalt an mindestens einem substituierten Thiadiazolyloxyessigsäureamid der Formel (I) gemäß Anspruch 1.

5. Verwendung von substituierten Thiadiazolyloxyessigsäureamiden der Formel (I) gemäß Anspruch 1 zur Bekämpfung von unerwünschtem Pflanzenwachstum.

6. Verfahren zur Herstellung von herbiziden Mitteln, dadurch gekennzeichnet, daß man substituierte Thiadiazolyloxyessigsäureamide der Formel (I) gemäß Anspruch 1 mit Streckmitteln und/oder oberflächenaktiven Mitteln vermischt.

7. Methylsulfonylthiadiazole der allgemeinen Formel (II)

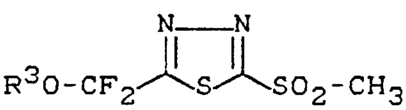

(II)

in welcher

$R^3$ für gegebenenfalls substituiertes Alkyl steht.

8. Methylthio-thiadiazole der allgemeinen Formel (IV)

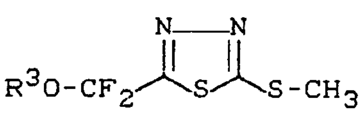

(IV)

in welcher

$R^3$ für gegebenenfalls substituiertes Alkyl steht.

## Claims

1. Substituted thiadiazolyloxyacetamides of the general formula (I)

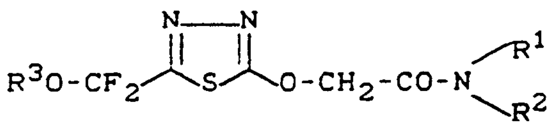

(I)

in which

$R^1$ represents hydrogen, $C_1$-$C_8$-alkyl which is optionally substituted by fluorine, chlorine, cyano or $C_1$-$C_4$-alkoxy, $C_2$-$C_8$-alkenyl which is optionally substituted by fluorine and/or chlorine, $C_2$-$C_8$-alkinyl or benzyl,

$R^2$ represents $C_1$-$C_8$-alkyl which is optionally substituted by fluorine, chlorine, cyano or $C_1$-$C_4$-alkoxy, $C_2$-$C_8$-alkenyl which is optionally substituted by fluorine and/or chlorine, $C_2$-$C_8$-alkinyl, $C_3$-$C_6$-cycloalkyl which is optionally substituted by chlorine and/or $C_1$-$C_3$-alkyl, $C_5$- or $C_6$-cycloalkenyl, benzyl which is optionally substituted by fluorine, chlorine and/or $C_1$-$C_4$-alkyl, phenyl which is optionally substituted by fluorine, chlorine, bromine, iodine, cyano, nitro, $C_1$-$C_4$-alkyl, trifluoromethyl, $C_1$-$C_4$-alkoxy and/or $C_1$-$C_4$-alkylthio, $C_1$-$C_8$-alkoxy which is optionally substituted by $C_1$-$C_4$-alkoxy, or $C_3$-$C_4$-alkenyloxy, or

$R^1$ and $R^2$ together with the nitrogen atom to which they are bonded form a saturated or unsaturated five- to seven-membered nitrogen heterocycle which is optionally monosubstituted to trisubstituted by $C_1$-$C_3$-alkyl and which is optionally benzo-fused, and

$R^3$ represents $C_1$-$C_6$-alkyl which is optionally substituted by halogen, $C_1$-$C_4$-alkoxy, $C_1$-$C_4$-alkylsulphonyl, phenyl, phenoxy or phenyl-$C_1$-$C_4$-alkoxy, the phenyl component being optionally substituted y halogen, $C_1$-$C_4$-alkyl, trifluoromethyl and/or $C_1$-$C_4$-alkoxy.

2. Compounds of the formula (I) according to Claim 1, characterised in that, in this formula,

$R^1$ represents $C_1$-$C_4$-alkyl, allyl or propargyl,

$R^2$ represents $C_1$-$C_6$-alkyl, $C_1$-$C_2$-alkoxy-$C_1$-$C_2$-alkyl, allyl, propargyl, cyclopentyl, cyclohexyl, cyclohexenyl, benzyl, phenyl (which is optionally substituted by fluorine, chlorine, bromine, cyano, nitro, methyl, ethyl, trifluoromethyl, methoxy and/or ethoxy), $C_1$-$C_6$-alkoxy or $C_1$-$C_2$-alkoxy-$C_1$-$C_2$-alkoxy, or

$R^1$ and $R^2$ together with the nitrogen atom to which they are bonded represent piperidinyl which is optionally monosubstituted to trisubstituted by methyl and/or ethyl, pyrrolidinyl which is optionally monosubstituted or disubstituted by methyl and/or ethyl, perhydroazepinyl or 1,2,3,4-tetrahydroquinolinyl, and

$R^3$ represents methyl, ethyl, propyl, isopropyl, butyl, isobutyl, sec-butyl, tert-butyl, trifluoroethyl, trichloroethyl, methoxyethyl, ethoxyethyl, methylsulphonylethyl, ethylsulphonylethyl, benzyl, chlorobenzyl, phenylethyl or benzyloxyethyl.

3. Process for the preparation of substituted thiadiazolyloxyacetamides of the formula (I) according to Claim 1, characterised in that methylsulphonylthiadiazoles of the general formula (II)

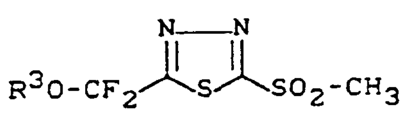

(II)

in which

$R^3$ has the meaning given to Claim 1,

are reacted with hydroxyacetamides of the general formula (III)

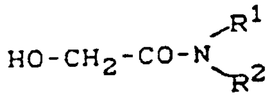

(III)

in which

$R^1$ and $R^2$ have the meanings given in Claim 1,

if appropriate in the presence of a diluent, if appropriate in the presence of an acid-binding agent and if appropriate in the presence of a catalyst.

4. Herbicidal agents, characterised in that they contain at least one substituted thiadiazolyloxyacetamide of the formula (I) according to Claim 1.

5. Use of substituted thiadiazolyloxyacetamides of the formula (I) according to Claim 1 for combating undesired plant growth.

6. Process for the preparation of herbicidal agents, characterised in that substituted thiadiazolyloxyacetamides of the formula (I) according to Claim 1 are mixed with extenders and/or surface-active agents.

7. Methylsulphonylthiadiazoles of the general formula (II)

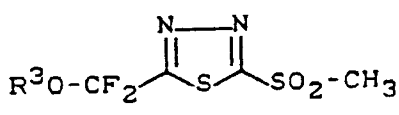

(II)

in which

$R^3$ represents optionally substituted alkyl.

8. Methylthio-thiadiazoles of the general formula (IV)

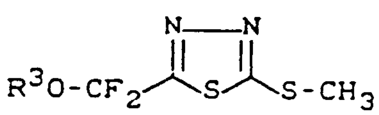

(IV)

in which

$R^3$ represents optionally substituted alkyl.

## Revendications

1. Thiadiazolyloxyacétamides substitués de formule générale (I)

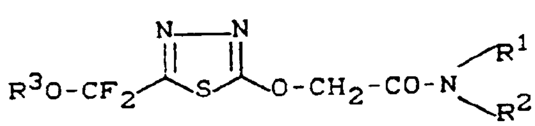

(I)

dans laquelle

$R^1$ représente l'hydrogène, un groupe alkyle en $C_1$-$C_8$ éventuellement substitué par le fluor, le chlore, un groupe cyano ou alcoxy en $C_1$-$C_4$, un groupe alcényle en $C_2$-$C_8$ éventuellement substitué par le fluor et/ou le chlore, un groupe alcynyle en $C_2$-$C_8$ ou un groupe benzyle,

$R^2$ représente un groupe alkyle en $C_1$-$C_8$ éventuellement substitué par le fluor, le chlore, un groupe

cyano ou alcoxy en $C_1$-$C_4$, un groupe alcényle en $C_2$-$C_8$ éventuellement substitué par le fluor et/ou le chlore, un groupe alcynyle en $C_2$-$C_8$, un groupe cycloalkyle en $C_3$-$C_6$ éventuellement substitué par le chlore et/ou un groupe alkyle en $C_1$-$C_3$, un groupe cycloalcényle en $C_5$ ou $C_6$, un groupe benzyle éventuellement substitué par le fluor, le chlore et/ou un groupe alkyle en $C_1$-$C_4$, un groupe phényle éventuellement substitué par le fluor, le chlore, le brome, l'iode, par un groupe cyano, nitro, alkyle en $C_1$-$C_4$, trifluorométhyle, alcoxy en $C_1$-$C_4$ et/ou alkylthio en $C_1$-$C_4$, un groupe alcoxy en $C_1$-$C_8$ éventuellement substitué par un groupe alcoxy en $C_1$-$C_4$, ou un groupe alcényloxy en $C_3$-$C_4$, ou

$R^1$ et $R^2$ forment ensemble avec l'atome d'azote auquel ils sont liés un hétérocycle azoté saturé ou insaturé à cinq à sept chaînons, éventuellement substitué une à trois fois par un groupe alkyle en $C_1$-$C_3$, et éventuellement accolé à un groupe benzénique et

$R^3$ représente un groupe alkyle en $C_1$-$C_6$ éventuellement substitué par un halogène, un groupe alcoxy en $C_1$-$C_4$, alkylsulfonyle en $C_1$-$C_4$, phényle, phénoxy ou phényl-alcoxy en $C_1$-$C_4$, le composant phényle étant éventuellement substitué par un halogène, un groupe alkyle en $C_1$-$C_4$, un groupe trifluorométhyle et/ou un groupe alcoxy en $C_1$-$C_4$.

**2.** Composés de formule (I) selon la revendication 1, caractérisés en ce que

$R^1$ signifie alkyle en $C_1$-$C_4$, allyle ou propargyle,

$R^2$ signifie alkyle en $C_1$-$C_6$, alcoxy en $C_1$-$C_2$-alkyle en $C_1$-$C_2$, allyle, propargyle, cyclopentyle, cyclohexyle, cyclohexényle, benzyle, phényle (éventuellement substitué par le fluor, le chlore, le brome, un groupe cyano, nitro, méthyle, éthyle, trifluorométhyle, méthoxy et/ou éthoxy), alcoxy en $C_1$-$C_6$ ou alcoxy en $C_1$-$C_2$-alcoxy en $C_1$-$C_2$, ou

$R^1$ et $R^2$ représentent ensemble avec l'atome d'azote auquel ils sont liés un groupe pipéridinyle éventuellement substitué une à trois fois par un groupe méthyle et/ou éthyle, un groupe pyrrolidinyle éventuellement substitué une ou deux fois par un groupe méthyle et/ou éthyle, un groupe perhydroazépinyle ou un groupe 1,2,3,4-tétrahydroquinolinyle, et

$R^3$ signifie méthyle, éthyle, propyle, isopropyle, butyle, isobutyle, butyle-sec., butyle tert., trifluoroéthyle, trichloroéthyle, méthoxyéthyle, éthoxyéthyle, méthylsulfonyléthyle, éthylsulfonyléthyle, benzyle, chlorobenzyle, phényléthyle ou benzyloxyéthyle.

**3.** Procédé pour fabriquer des thiadiazolyloxyacétamides substitués de formule (I) selon la revendication 1, caractérisé en ce que l'on fait réagir des méthylsulfonylthiadiazoles de formule générale (II)

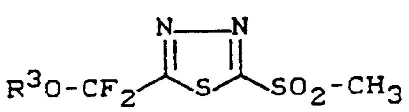

(II)

dans laquelle

$R^3$ possède la signification décrite dans la revendication 1

avec des hydroxyacétamides de formule générale (III)

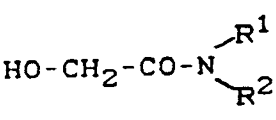

(III)

dans laquelle

$R^1$ et $R^2$ ont les significations décrites dans la revendication 1,

éventuellement en présence d'un diluant, éventuellement en présence d'un liant d'acide et éventuellement en présence d'un catalyseur.

**4.** Agents herbicides caractérisés en ce qu'il contiennent au moins un thiadiazolyloxyacétamide substitué de formule (I) selon la revendication 1.

**5.** Utilisation des thiadiazolyloxyacétamides substitués de formule (I) selon la revendication 1 pour lutter contre les développements indésirables de plantes.

**6.** Procédé pour la fabrication d'agents herbicides, caractérisé en ce que l'on mélange des thiadiazolyloxyacétamides substitués de formule (I) selon la revendication 1 avec des agents d'allongement et/ou des agents tensioactifs.

7. Méthylsulfonylthiadiazoles de formule générale (II)

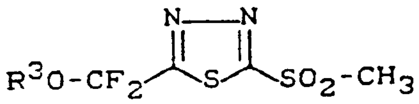

(II)

dans laquelle

$R^3$ représente un groupe alkyle éventuellement substitué.

8. Méthylthiothiadiazoles de formule générale (IV)

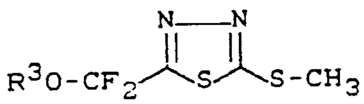

(IV)

dans laquelle

$R^3$ représente un groupe alkyle éventuellement substitué.